# EUROPEAN PATENT SPECIFICATION

(11) **EP 1 698 362 B1**
(45) Date of publication and mention of the grant of the patent: **30.03.2011**
(21) Application number: 06010943.6
(22) Date of filing: 13.04.2004
(51) Int. Cl.: A61M 1/16, A61M 1/34

(54) **Blood treatment machine and unit**
Blutbehandlungsmaschine sowie Blutbehandlungseinheit
Machine et unité de traitement du sang

(30) Priority: 03.10.2003 IT TO20030785
(43) Date of publication of application: 06.09.2006
(62) Divisional of application: 04101504.1
(73) Proprietor: Medical Service S.r.l., 84131 Salerno (IT)
(72) Inventor: Pasqualini, Gianni, I-45100 Rovigo (IT)
(74) Representative: Barberi, Vittorio

(56) References cited:
- EP-A- 0 705 610
- EP-A- 0 890 368
- US-A1- 2002 077 581

## Description

The present invention relates to a blood treatment machine and unit.

More specifically, the present invention relates to a machine designed to permit respiration support therapy and/or extrarenal purification therapy, such as CRRT (Continuous Renal Replacement Therapy), of a patient, to which application the following description refers purely by way of example. As is known, extrarenal blood purification systems or so-called "haemofiltration systems" exist which provide for purifying the patient's blood of waste liquids and/or soluble substances which accumulate in the blood for pathological and/or surgical reasons, and/or as a result of substances administered to the patient, thus performing the functions normally carried out by healthy kidneys in correct working condition.

Haemofiltration systems of the above type normally comprise a blood purification circuit, along which the patient's blood is fed to add any necessary supplements and/or to purify it of any toxic solutes, and are connected to the patient by two feed conduits or catheters: one for supplying the non-purified venous blood of the patient to the purification circuit, and the other for feeding the purified blood into the patient's vein.

The purification circuit normally comprises a pump connected to a first catheter by a conduit for receiving the patient's blood, and which pumps the patient's blood along the purification circuit; a unit for adding an anticoagulant to the blood; a heating and supplementing unit for adding a liquid supplement to the blood and heating the liquid supplement to a predetermined temperature; and a blood purifying filter or so-called haemofilter, which is connected to the second catheter and provides for eliminating any toxic elements in the blood before it is fed back to the patient's body. In certain patients in particularly critical condition, extrarenal purification therapy must very often be accompanied by respiration support therapy, by which the oxygen concentration of the patient's blood is supplemented by removing excess carbon dioxide (CO₂) from the blood.

EP-0 705 610 A relates to extracorporeal circulation systems intended to take over the functions of the lungs and the circulation system during heart surgery requiring a heart-lung by-pass. This document shows an extracorporeal circulation system which comprises, among other devices, an oxygenator and a haemo-filten.

Systems of the above type, however, though providing for highly effective extrarenal blood purification, fail to provide for simultaneous respiration support therapy, so that the patient, during extrarenal purification therapy, must be ventilated by non-physiological means, with obvious drawbacks and discomfort to the patient.

It is an object of the present invention to provide a machine designed to permit simultaneous extrarenal purification therapy and respiration support therapy of a patient.

Besides, with the present invention is possible, during blood purification, to remove the plasma water from the blood and feeds it to a CO₂ removing device, thus advantageously ensuring the blood in CO₂ removing device is diluted sufficiently.

According to the present invention, there is provided a blood treatment unit as claimed in Claim 1.

A non-limiting embodiment of the present invention will be described by way of example with reference to the accompanying drawings, in which:
- Figure 1 shows, schematically, a blood treatment machine comprising a blood treatment unit in accordance with the teachings of the present invention;
- Figure 2 shows a schematic lateral section of the Figure 1 blood treatment unit;
- Figure 3 shows a schematic side view, with parts in section, of a variation of the Figure 2 blood treatment unit;
- Figure 4 shows, schematically a variation of the Figure 1 machine.

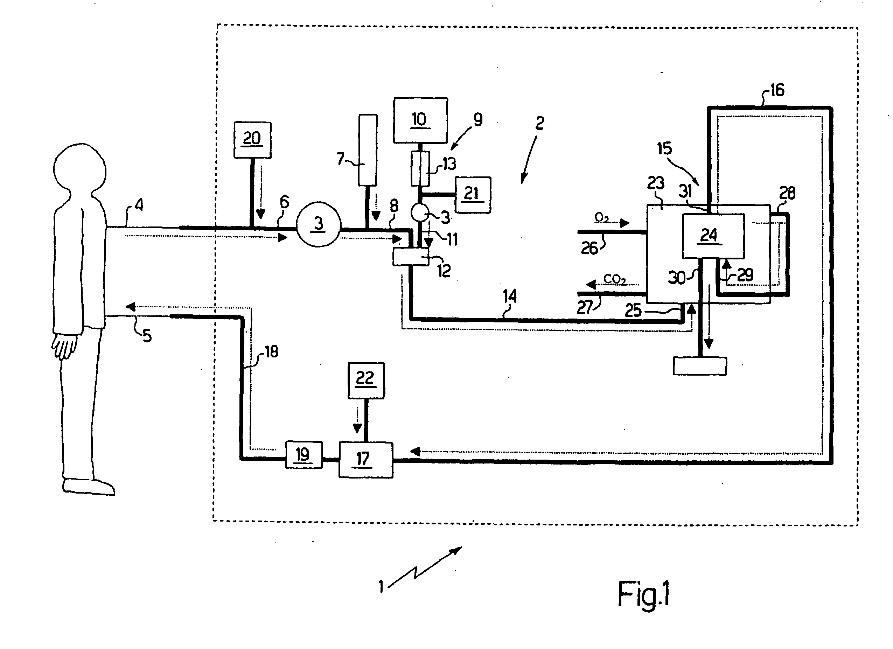

Number 1 in Figure 1 indicates as a whole a machine designed to permit simultaneous extrarenal blood purification therapy, i.e. haemofiltration, and respiration support therapy of a patient.

Machine 1 substantially comprises a blood purification circuit 2, along which the patient's blood is fed to add liquid supplements and to purify it of any waste solutes and/or toxic elements; and one or more flow pumps 3 to maintain a given blood flow along blood purification circuit 2.

Blood purification circuit 2 is connected to the patient's cardiocirculatory system by two blood feed conduits or catheters, one of which, indicated hereinafter by 4, receives and supplies blood from the patient's vein to blood purification circuit 2, while the other, indicated hereinafter by 5, is inserted inside a vein to feed the purified blood back into the patient's cardiocirculatory system.

In the example shown, blood purification circuit 2 comprises an inlet conduit 6 connecting catheter 4 to flow pump 3; and a unit 7 for adding an anticoagulant to the blood, and which is connected to an outlet conduit 8 of flow pump 3 to add the anticoagulant to the blood flow. In the example shown, unit 7 may be defined by a tank 7 containing anticoagulant such as heparin or similar.

With reference to Figure 1, blood purifying circuit 2 also comprises a supplementing unit 9 for appropriately heating a liquid supplement or infusion to a given temperature and then adding it to the blood flowing in purification circuit 2. In the example shown, supplementing unit 9 comprises a tank 10 containing the liquid supplement or infusion; a conduit 11 connecting tank 10 to a connecting member 12 via a flow pump 3; and a heating device 13 connected to conduit 11 between flow pump 3 and tank 10 to heat the liquid supplement or infusion before it is mixed with the blood.

In addition to conduit 11, connecting member 12 is also connected at the inlet to the outlet conduit 8 of tank 7, and provides for "mixing" the heated liquid supplement or infusion with the blood, and feeding the blood to an outlet conduit 14.

Purification circuit 2 also comprises a blood treatment unit 15 for both removing excess CO2 from the blood, thus performing respiration support therapy, and ultrafiltering the blood of waste substances and/or liquids, thus performing extrarenal purification therapy.

In the example shown, treatment unit 15 is connected at the inlet to the outlet conduit 14 of connecting member 12 to receive the mixed blood for CO2 removal and purification, and is connected at the outlet, by a conduit 16, to a blood collecting vessel 17, in turn connected at the outlet to catheter 5 by a connecting conduit 18. In the example shown, blood collecting vessel 17 may be defined, for example, by a known venous blood collecting tank not described in detail.

An air detecting device 19 is fitted along connecting conduit 18, i.e. downstream from blood collecting vessel 17, to remove from the blood any air bubbles which, if transmitted to the patient, could produce an embolism or other serious consequences.

Preferably, though not necessarily, purification circuit 2 also comprises a pressure gauge 20 connected to inlet conduit 6, upstream from flow pump 3, to measure the "intake" pressure of the blood from the patient; a pressure gauge 21 connected to conduit 11 to measure the pressure of the liquid supplement from tank 10; and a pressure gauge 22 cooperating with blood collecting vessel 17 to measure the pressure of the blood to be fed back into the patient's cardiocirculatory system.

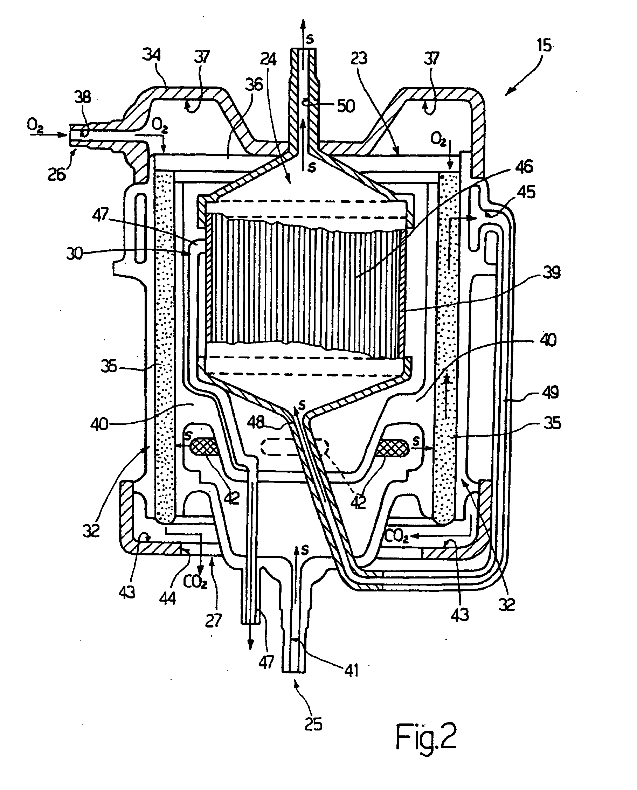

Treatment unit 15 comprises a CO2 removing device 23 and a filter 24, which, as shown schematically in Figures 1 and 2, are integrated to form one body, and provide respectively for eliminating a given amount of CO2 from the blood, and ultrafiltering the blood of waste liquids and substances and/or toxic substances.

In the example shown schematically in Figure 1, CO₂ removing device 23 is connected to filter 24 so that filter 24 is substantially integrated in CO2 removing device 23. More specifically, CO2 removing device 23 houses filter 24, and is connected upstream from filter 24 with respect to the blood flow direction along purifying circuit 2.

As shown in Figure 1, CO₂ removing device 23 has an inlet 25 connected to conduit 14 to receive the blood for CO₂ removal and purification; an inlet 26 connectable to an oxygen cylinder or tank (not shown) to receive a predetermined amount of oxygen by which to remove CO₂ from the blood; an exhaust outlet 27 from which CO₂ removed from the blood is expelled; and an outlet 28 for the blood deprived of CO₂.

Filter 24 is connected downstream from CO₂ removing device 23, and has an inlet 29 connected to and supplied with blood deprived of CO₂ by outlet 28 of CO2 removing device 23; an outlet 30 for expelling the ultrafiltrate containing the waste liquids and toxic and/or waste substances filtered from the blood; and an outlet 31 supplying to conduit 16 the blood purified and deprived of CO₂.

With reference to Figure 2, as stated, CO₂ removing device 23 and filter 24 defining treatment unit 15 are advantageously integrated to form one body.

More specifically, treatment unit 15 comprises two airtight containers or casings made of rigid material, such as polycarbonate or similar, and arranged one inside the other to define a seat 32 in between.

The outer casing, indicated 34, is tubular, preferably, though not necessary, in the form of a cylinder or parallelepiped, and houses, in the gap defined by seat 32, a bundle of membranes 35, through and/or over which oxygen and a stream of pressurized blood flow in use, so that the haemoglobin in the blood releases CO₂ and simultaneously acquires oxygen to remove CO₂ from the blood.

Membranes 35 may be defined by a number of sheets or hollow fibres of polypropylene or similar material, and are arranged parallel to preferably occupy a central portion of seat 32.

The hollow fibres may obviously be arranged in any manner inside seat 32, e.g. crossed and/or wound to form a predetermined oxygen-blood exchange surface of, say, roughly 0,6-0,7 m².

In the Figure 2 example, the top of the bundle of membranes 35 is connected rigidly to the top end of outer casing 34 by a supporting plate 36, which extends outwards to define, with the inner wall of the top end, a top gap 37 communicating with the outside of outer casing 34 via an oxygen inlet channel 38 defining inlet 26 of CO₂ removing device 23.

More specifically, as shown clearly in Figure 2, oxygen inlet channel 38 is formed in the lateral wall of outer casing 34, and feeds the oxygen through gap 37 downwards (arrow O₂), i.e. towards membranes 35.

In the gap between membranes 35 and the inner casing, hereinafter indicated 39, a bell-shaped member 40 is fitted beneath plate 36, is closed at the top by plate 36, and has, at the opposite end, an inlet channel 41 defining inlet 25 of CO2 removing device 23. In the example shown, inlet channel 41 extends through the centre of the bottom end of outer casing 34, and provides for feeding the blood for CO2 removal and purification (arrow S) into bell-shaped member 40.

At the bottom end of the bundle of membranes 35, bell-shaped member 40 has a number of through holes 42, through which, in use, the pressurized blood inside bell-shaped member 40 is pushed out towards membranes 35.

The bottom end of outer casing 34 and the bottom portion of bell-shaped member 40 define a bottom gap 43 communicating with membranes 35 and for receiving the CO₂ released by the haemoglobin in the blood during oxygenation. As shown clearly in the Figure 2 example, bottom gap 43 communicates externally (arrow CO₂) via a CO₂ exhaust channel 44 formed in the bottom end of outer casing 34 and defining outlet 27 of CO₂ removing device 23.

With reference to Figure 2, at the top of membranes 35, beneath plate 36, the lateral wall of outer casing 34 has an outlet channel 45 for the blood from CO2 removing device 23, and from which CO2 has been removed.

Inner casing 39 is tubular, preferably, though not necessarily, in the form of a cylinder or parallelepiped, and is fixed rigidly by one end to a corresponding end of outer casing 34. More specifically, in the Figure 2 example, inner casing 39 is housed inside bell-shaped member 40, and is connected rigidly at the top end to the top end of outer casing 34.

Inner casing 39 in turn houses a number of highly permeable, semipermeable membranes 46 which, when subjected to hydrostatic pressure, provide for ultrafiltrating the blood to eliminate toxic elements dissolved in the blood.

More specifically, in the Figure 2 example, semipermeable membranes 46 are preferably, though not necessarily, defined by a bundle of hollow fibres made of highly permeable polysulphone, arranged substantially parallel, and along which the blood for filtering flows in use.

As ultrafiltration removes a considerable amount of liquid from the blood, inner casing 39 has a drain channel 47 for expelling the liquids and toxic elements removed from the blood. More specifically, drain channel 47 defines outlet 30 of filter 24, and, as shown in the Figure 2 example, extends from a lateral wall of and at the top end of inner casing 39, and partly through bell-shaped member 40 to come out inside outer casing 34.

Inner casing 39 also comprises a blood inlet channel 48, which is connected by a connecting conduit 49 to outlet channel 45 of outer casing 34, from which it receives the blood deprived of CO₂.

As shown clearly in the Figure 2 example, blood inlet channel 48 is formed in the bottom end of inner casing 39; and connecting conduit 49 extends from blood inlet channel 48, along an inner portion of bell-shaped member 40, and preferably, though not necessarily, comes out outside outer casing 34 to connect up with outlet channel 45 of outer casing 34.

At the top end of inner casing 39, opposite blood inlet channel 48, a blood outlet channel 50 is provided for the blood purified and deprived of CO₂. In the Figure 2 example, blood outlet channel 50 defines outlet 31 of filter 24, extends through the centre of the top end of outer casing 34, and comes out outside outer casing 34.

When machine 1 is operated, flow pump 3 receives and feeds the blood from catheter 4 to connecting member 12, while at the same time unit 7 adds anticoagulant to the blood; and connecting member 12 mixes the blood with the liquid supplement supplied by supplementing unit 9, and feeds the blood to treatment unit 15.

At treatment unit 15, the blood flows along inlet channel 41, and is forced by pressure inside bell-shaped member 40 and out through holes 42 towards the bottom end of the bundle of membranes 35.

The pressurized blood forced upwards flows over and/or through membranes 35, while, at the opposite end, the oxygen flowing in from channel 38 and through top gap 37 is directed towards the top of membranes 35, flows through and/or over membranes 35, and gradually downwards in the opposite direction to the blood flow. As it flows through membranes 35, the haemoglobin in the blood acquires oxygen and releases excess CO₂, which flows to the bottom of outer casing 34, through bottom gap 43, and out through exhaust channel 44.

CO₂ removal from the blood is completed by the time the blood reaches the top of the bundle of membranes 35, where outlet channel 45 feeds the flow of the blood deprived of CO₂ along conduit 49 to inlet channel 48 of inner casing 39, inside which purification takes place.

The incoming blood from inlet channel 48 encounters and flows through semipermeable membranes 46, which separate the blood from the waste substances and/or toxic substances and surplus liquids, which are expelled from inner casing 39 along drain channel 47; and the pressurized blood is forced gradually towards outlet channel 50, from which it flows out purified.

At this point, the blood flows through blood collecting vessel 17 and air detecting device 19, and back into the patient's cardiocirculatory system along catheter 5.

Machine 1 as described above is extremely advantageous, in that, integrating CO₂ removing device 23 and filter 24 in one body, i.e. one treatment unit 15, permits simultaneous respiration support and extrarenal purification therapy, thus eliminating the need for non-physiological ventilation of the patient, with obvious benefits to the patient.

It should be pointed out that, besides reducing the size of treatment unit 15, integration of CO₂ removing device 23 and filter 24 in one treatment unit 15 also simplifies connection of the various inlet/outlet conduits to purification circuit 2, so that treatment unit 15 can be assembled faster, with less likelihood of the circuit being connected wrongly.

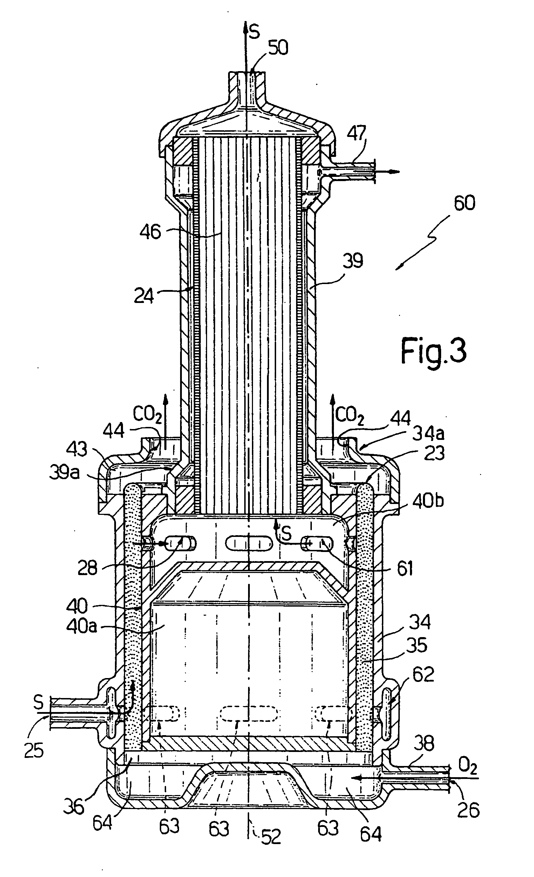

The Figure 3 embodiment relates to a treatment unit 60 similar to treatment unit 15, and the component parts of which are indicated, where possible, using the same reference numbers as for the corresponding parts of treatment unit 15.

Treatment unit 60 differs from treatment unit 15 by CO₂ removing device 23 being connected to filter 24 so that filter 24 projects outwards of, as opposed to being housed completely inside, CO₂ removing device 23.

More specifically, as opposed to being housed completely inside outer casing 34 (containing CO₂ removing device 23), inner casing 39 containing filter 24 projects outwards and upwards from outer casing 34.

In the configuration shown in the Figure 3 example, inner casing 39 is connected to the top of outer casing 34, and extends upwards coaxially with the longitudinal axis 52 of treatment unit 60. More specifically, the base portion 39a of inner casing 39 is fixed rigidly, preferably heat sealed, to the top portion 34a of outer casing 34.

As shown in Figure 3, bell-shaped member 40 of treatment unit 60 has no through holes 42 at the bottom, and is divided into two separate portions : a bottom portion 40a closed hermetically, and the outer lateral surface of which rests on membranes 35; and a top portion 40b having a number of through holes 61, each defining outlet 28 and through which the blood deprived of CO2 flows from CO2 removing device 23 to filter 24. Top portion 40b is also connected to filter 24 by one or more conduits (not shown) by which the blood deprived of CO2 flows into filter 24.

Inlet 25 of CO₂ removing device 23 is connected to an annular conduit 62 formed in the outer lateral wall of outer casing 34, which has a number of inner through holes 63 facing, and through which the pressurized blood flows towards, membranes 35.

In addition to the above, the bottom end of the bundle of membranes 35 is connected rigidly to the bottom end of outer casing 34 by supporting plate 36, which extends outwards to define, with the inner wall of the bottom end, a bottom gap 64 communicating with the outside of outer casing 34 via oxygen inlet channel 38 defining inlet 26 of CO₂ removing device 23.

In actual use, the blood to be filtered flows into CO₂ removing device 23 through inlet 25, into annular conduit 62, and out through holes 63 so as to flow under pressure to the bottom of the bundle of membranes 35. The pressurized blood forced upwards flows over and/or through membranes 35, while, at the same time, the pressurized oxygen flowing in from channel 38 and through bottom gap 64 is directed towards the bottom of the bundle of membranes 35, flows through membranes 35, and gradually upwards in the same direction as the blood flow. As the blood and oxygen flow through hollow fibres 35, the haemoglobin in the blood acquires oxygen and releases excess CO₂, which flows to the top of outer casing 34, through a top gap 43, and out through exhaust channel 44.

CO₂ removal is completed by the time the blood reaches the top of the bundle of membranes 35, where through holes 61 feed the flow of the blood deprived of CO₂ along respective conduits (not shown) to the inlet (not shown) of inner casing 39, inside which filtration takes place.

The blood then flows through semipermeable membranes 46, which separate the blood from the waste substances and/or toxic substances and surplus liquids, which are expelled from inner casing 39 along drain channel 47; and the pressurized blood is forced gradually towards outlet channel 50, from which it flows out purified.

According to the present invention, treatment units 15 and 60 described above may be used to advantage in a machine for respiration support therapy only, e.g. in patients with no kidney malfunction; in which case, the ultrafiltrate from drain channel 47 of CO₂ removing device 23 is obviously defined substantially by a dilution liquid, such as plasma water, and therefore contains no waste and/or toxic substances.

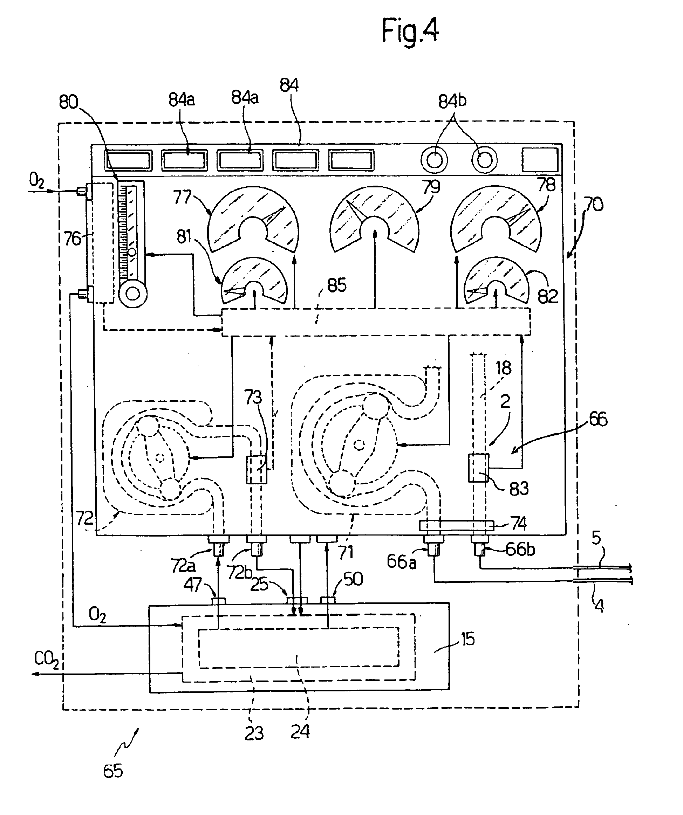

Figure 4 shows one possible application of a treatment unit 15 (or 60) in a machine 65 for removing CO₂ from the blood, which therefore provides solely for respiration support therapy of a patient with no kidney malfunction.

Machine 65 substantially comprises a blood flow circuit 66 (shown partly in Figure 4) connectable to the patient's cardiocirculatory system by catheters 4 and 5 for receiving blood from the patient and feeding it back, purified, to the patient; a treatment unit 15 (or 60) connected to blood flow circuit 66 to perform respiration support therapy; and an electronic control device 70 for coordinating the process for removing CO2 from the blood and performed by treatment unit 15, and displaying a series of measuring parameters to monitor CO2 removal continuously.

More specifically, electronic control device 70 comprises at least two blood flow pumps connected along blood flow circuit 66 (shown partly) : a first flow pump, hereinafter indicated 71, which circulates the blood at a given flow rate along flow circuit 66 and through treatment unit 15; and a second flow pump, hereinafter indicated 72, which receives the ultrafiltrate from drain channel 47, and supplies it to treatment unit 15 to appropriately dilute the blood in CO₂ removing device 23.

As stated, the ultrafiltrate from drain channel 47 of filter 24, coming from a patient with healthy kidneys, substantially comprises a dilution liquid defined by plasma water (with no waste and/or toxic substances), so that treatment unit 15 operates in exactly the same way as an CO₂ removing device. That is, during blood purification, filter 24 simply removes the plasma water from the blood and feeds it to the CO₂ removing device, thus advantageously ensuring the blood in CO₂ removing device 23 is diluted sufficiently.

In connection with the above, it should be pointed out that filter 24, by simply providing in this case for removing the plasma water from the blood, may conveniently be reduced, thus further reducing the overall size of treatment unit 15. More specifically, filter 24 is designed to remove enough plasma water from the blood to dilute the blood supplied to CO₂ removing device 23, thus eliminating the need to excessively "coagulate" the patient, while at the same time ensuring correct operation of membranes 35 even for prolonged periods (e.g. 24 hours).

It should also be pointed out that, in this case, besides drastically reducing blood flow resistance, thus reducing haemolysis, i.e. traumatic destruction of red blood cells, filter 24 also enables a sufficient pressure drop to be maintained in treatment unit 15 to prevent gas bubbles accidentally entering the blood, thus safeguarding the patient against gaseous embolisms.

With reference to Figure 4, pump 72 has an inlet 72a connected to drain channel 47 of filter 24, and an outlet 72b connected to inlet 25 of treatment unit 15; and pump 71 is located along flow circuit 66, which has an inlet 66a connected to catheter 4 to receive the blood for CO₂ removal, and an outlet 66b connected by a conduit to catheter 5, which is inserted inside a vein to feed the blood deprived of CO₂ back into the patient's cardiocirculatory system.

In the Figure 4 example, pumps 71 and 72 are defined by two peristaltic, e.g. roller, pumps, one of which provides for circulating blood at a higher flow rate than the other. In the example shown, pump 71 may be designed for a flow rate of preferably about 350 ml/minute, and pump 72 is designed for a flow rate of preferably about 53 ml/minute.

Electronic control device 70 also comprises a first pressure detecting device 73 for supplying an output signal relative to the pressure of the ultrafiltrate supplied to CO₂ removing device 23 to dilute the blood; and a second pressure detecting device 74 for supplying an output signal relative to the intake pressure of the blood from the patient, and an output signal relative to the feedback pressure of the blood to the patient's body.

Electronic control device 70 also comprises a detecting device 76, e.g. a flow gauge, for supplying an output signal relative to the oxygen flow rate to treatment unit 15.

With reference to Figure 4, electronic control device 70 also comprises an ultrafiltrate pressure display device 77; a display device 78 showing the feedback pressure of the blood to the patient's body; an intake blood pressure display device 79; a display device 80 showing the oxygen flow rate to treatment unit 15; a display device 81 showing the flow of pump 71, i.e. the blood flow absorbed by the patient; and a display device 82 showing the flow of pump 72, i.e. ultrafiltrate flow to treatment unit 15.

Preferably, though not necessarily, electronic control device 70 also comprises a display device (not shown) showing the fall in pressure in the blood flow from pump 71 to treatment unit 15.

Electronic control device 70 also comprises a sensor 83 preferably located along connecting conduit 18 of blood flow circuit 66, and for supplying an output signal relative to the presence of air or gas bubbles in the filtered feedback blood before it is fed back to the patient's body; and an acoustic/visual indicator device 84 for generating on command an acoustic/visual alarm signal to inform the operator of a possible malfunction of the machine and/or a patient hazard condition.

More specifically, in the Figure 4 example, acoustic/visual indicator device 84 comprises a number of displays 84a and/or acoustic indicators 84b, each indicating a corresponding hazard condition or malfunction, such as a sudden fall in blood pressure between pump 71 and treatment unit 15, intake or feedback blood pressure or flow outside a predetermined safety range, low oxygen supply to treatment unit 15, or the presence of air bubbles in the blood.

Electronic control device 70 also comprises a central control block 85 for receiving the measured ultrafiltrate pressure signal from first pressure detecting device 73, and displaying it on display device 77; receiving the measured intake and feedback pressure signals from second pressure detecting device 74, and displaying them on display devices 78 and 79 respectively; and receiving the oxygen flow rate signal from detecting device 76, and displaying it on display device 80.

Central control block 85 also calculates, instant by instant, the blood flow supplied by pump 71 and the ultrafiltrate flow supplied by pump 72, and displays them on display devices 81 and 82 respectively.

Central control block 85 also receives the signal from sensor 83, and disables pumps 71 and 72 immediately if air bubbles are detected in the blood. When this occurs, central control block 85 preferably activates acoustic/visual indicator device 84 to inform the operator of a system malfunction and/or patient hazard condition.

Central control block 85 also determines, instant by instant, whether the intake or feedback blood pressure and/or flow values, the ultrafiltrate flow and/or pressure value, and the pressure drop conform with predetermined conditions on the basis of respective safety thresholds or ranges.

If one or more conditions are not conformed with, and/or if air bubbles are detected, central control block 85 : disables pumps 71 and 72; cuts off blood feedback to the patient and oxygen supply to the treatment unit; and activates acoustic/visual indicator device 84 to inform the operator of a system malfunction and/or patient hazard condition. It should be pointed out that blood feedback to the patient and/or oxygen supply to the treatment unit may be cut off by central control block 85 commanding closure of a number of solenoid valves (not shown) installed along flow circuit 66 and at the oxygen inlet.

In a different embodiment not shown, machine 65 may be equipped with a treatment unit 15 in which CO₂ removing device 23 and filter 24 are separate, i.e. not integrated in one body. In which case, drain channel 47 of filter 24 is still connected via second pump 72 to inlet 25 of CO₂ removing device 23 to supply CO₂ removing device 23 with sufficient excess liquid filtered from the blood to ensure correct dilution of the during removal of CO₂ from the blood.

Clearly, changes may be made to the blood treatment machine and unit as described and illustrated herein without, however, departing from the scope of the present invention.

## Claims

1. A blood treatment unit (15; 60) for a respiration support therapy comprising:
- CO₂ removing means (23) having: a first inlet (25) for receiving a flow of blood for CO₂ removal, a first outlet (28) for the flow of blood deprived of CO₂, an other inlet (26) for receiving oxygen, and an exhaust outlet (27) from which CO₂ removed from the blood is expelled;
- filtering means (24), disposed downstream of said CO₂ removing means (23), having at least a first inlet (48) for receiving the flow of blood, at least a first outlet (50) for the flow of purified blood, and at least one drain channel (47) by which, in use, a diluting liquid obtained from the blood is expelled during purification of the blood; said drain channel (47) being connected to the said first inlet (25) of said CO₂ removing means (23) to supply said diluting liquid to the CO₂ removing means (23),
**characterized in that** it comprises a pump (72) having an inlet (72a) connected to said drain channel (47) and an outlet (72b) connected to said first Inlet (25) of said CO2 removing means (23).

2. A blood treatment unit according to claim 1, **characterized in that** said CO₂ removing means (23) and said filtering means (24) are integrated to form one body.

3. A blood treatment unit (15) according to claim 2, **characterized in that** said CO₂ removing means (23) comprise an inner seat (40) housing said filtering means (24).

## Patentansprüche

1. Eine Einheit zur Behandlung von Blut (15, 60) für die Unterstützungstherapie, welche umfasst:
- Mittel zur Entfernung von CO₂ (23) mit folgenden Eigenschaften: ein erster Zugang (25) zur Aufnahme der Blutflusses für die Entfernung von CO2, ein erster Ausgang (28) für den Abfluss des CO₂-freien Blutes, ein weiterer Zugang (26) für den Zufluss von Sauerstoff, und ein Ausgang für den Abfluss (27), über den das aus dem Blut entfernte CO₂ ausgeschieden wird;
- Mittel zum Herausfiltern (24), die unterhalb der besagten Mittel zur Entfernung des CO₂ (23) angeordnet sind und mindestens über einen ersten Zugang (48) für den Zufluss des Blutes, mindestens einen ersten Ausgang (50) für den Abfluss des gereinigten Blutes und mindestens einen Drainagekanal (47) verfügen, über den bei Gebrauch eine Verdünnungsflüssigkeit, die aus dem Blut erhalten wird, das während der Reinigung des Blutes ausgeschieden wird; der besagte Drainagekanal (47), der mit dem besagten ersten Ausgang (25) der besagten Mittel zur Entfernung von CO₂ (23) verbunden ist, um den besagten Mitteln für die Entfernung von CO₂ (23) die besagte Verdünnungsflüssigkeit für die Entfernung von CO₂ (23) zuzuführen,
**dadurch gekennzeichnet, dass** sie eine Pumpe (72) mit einem Eingang (72a), der an den besagten Drainagekanal (47) angeschlossen ist, und einem Ausgang (72b), der an den besagten ersten Eingang (25) der besagten Mittel zur Entfernung von CO₂ angeschlossen ist, enthält.

2. Eine Einheit zur Behandlung des Bluts nach Anspruch 1, **dadurch gekennzeichnet, dass** die Mittel für die Entfernung von CO₂ (23) und besagte Mittel zum Herausfiltern (24) so integriert sind, dass sie einen einzigen Körper bilden.

3. Eine Einheit zur Behandlung von Blut (15) nach Anspruch 2, **dadurch gekennzeichnet, dass** die besagten Mittel zur Entfernung von CO₂ (23) eine interne Stelle (40) zur Anbringung der besagten Mittel zum Herausfiltern (24) besitzt.

## Revendications

1. Une unité de traitement du sang (15, 60) pour une thérapie de soutien respiratoire comprenant:
- des moyens pour le déplacement de CO₂ (23) ayant : une première entrée (25) pour recevoir un flux de sang pour l'élimination de CO₂, une première sortie (28) pour le flux de sang privé de CO₂, une autre entrée (26) pour recevoir de l'oxygène, et une sortie de décharge (27) à partir de laquelle le CO₂ éliminée du sang est expulsé;
- des moyens de filtrage (24), disposé en aval desdites moyens pour le déplacement de CO₂ (23), ayant au moins une première entrée (48) pour recevoir le flux de sang, au moins une première sortie (50) pour le flux du sang purifié, et au moins un canal de drainage (47) par lequel, en cours d'utilisation, un liquide de dilution obtenu à partir du sang est expulsé lors de la purification du sang; ledit canal de drainage (47) étant reliée à ladite première entrée (25) desdites moyens pour le déplacement de CO₂ (23) pour alimenter ledit liquide de dilution aux moyens pour le déplacement de CO₂ (23),
**caractérisée en ce qu'**elle comprend une pompe (72) ayant une entrée (72a) reliée à ledit canal de drainage (47) et une sortie (72b) reliée à ladite première entrée (25) de lesdites moyens pour le déplacement de CO₂ (23).

2. Une unité de traitement du sang selon la revendication 1, **caractérisée en ce que** lesdits moyens pour le déplacement de CO₂ (23) et lesdits moyens de filtrage (24) sont intégrés pour former un seul corps.

3. Une unité de traitement du sang (15) selon la revendication 2, **caractérisée en ce que** lesdits moyens pour le déplacement de CO₂ (23) comprennent un logement intérieur (40) pour le logement desdits moyens de filtrage (24).
